# EUROPEAN PATENT SPECIFICATION

(11) **EP 3 808 335 B1**
(45) Date of publication and mention of the grant of the patent: **22.05.2024**
(21) Application number: 19382904.1
(22) Date of filing: 15.10.2019
(51) Int. Cl.: A61K 8/9789, A61K 36/185, A61Q 19/08, A61K 36/746

(54) **COMPOSITIONS FOR TOPICAL ADMINISTRATION**
ZUSAMMENSETZUNGEN ZUR TOPISCHEN VERABREICHUNG
COMPOSITIONS POUR ADMINISTRATION TOPIQUE

(43) Date of publication of application: 21.04.2021
(73) Proprietor: FAES FARMA, S.A., 48940 Leioa - Vizcaya (ES)
(72) Inventor: HERNÁNDEZ HERRERO, Gonzalo, E-48940 Leioa - Vizcaya (ES); BLANCO FUENTE, Haydee Divina, E-48940 Leioa - Vizcaya (ES); GOÑI DE CERIO, Felipe, E-48170 Zamudio - Vizcaya (ES); GÓMEZ FERNÁNDEZ, Paloma, E-48170 Zamudio - Vizcaya (ES); JANÉ FONT, Albert, E-08221 Terrassa (ES); EXPÓSITO TARRÉS, Óscar, E-08221 Terrassa (ES)
(74) Representative: ABG Intellectual Property Law, S.L.

(56) References cited:
- WO-A1-2016/166047
- WO-A2-2016/120713
- ES-A1- 2 579 385
- FR-A1- 2 864 446
- US-A1- 2007 286 910
- SUGIYAMA MUNETAKA: "Historical review of research on plant cell dedifferentiation", JOURNAL OF PLANT RESEARCH, TOKYO, JP, vol. 128, no. 3, 1 March 2015 (2015-03-01) , pages 349-359, XP035491567, ISSN: 0918-9440, DOI: 10.1007/S10265-015-0706-Y [retrieved on 2015-03-01]

## Description

### FIELD OF THE INVENTION

The present invention relates to a cosmetic and/or pharmaceutical composition for topical administration, more particularly, to a composition optimized for ameliorating, treating and/or repairing skin disorders related to inflammation such as atopic dermatitis, seborrheic dermatitis, psoriasis, acne, and others.

### BACKGROUND

The stratum corneum, or epidermis, is the top layer of the skin and is known for its barrier function. It forms a protective covering for the skin and controls the flow of water and substances in and out of the skin. The lower level of the skin is known as the dermis. Dermis is the layer which provides the strength, elasticity and the thickness to the skin.

The skin is often prone to external aggressions by soaps, emulsifier-based cosmetics, hot water, or organic solvents. Furthermore, the skin is subject to deterioration through dermatological disorders, environmental abuse (wind, UV rays, pollution, heat, over-dieting, and others) or through the normal aging process (chronoaging). The thickness of the dermal layer is reduced due to aging, thus causing the skin to slacken. This is believed to be partially responsible for the formation of wrinkles.

Plant extracts and the use of parts of plants, such as leaves, fruits, flowers, stems, bark, inflorescences and roots for cosmetic and medical applications are known since ancient times. Products derived therefrom may be, e.g., essential oils, fibers, starch, flavors, coloring matters, antibiotics, proteins, phenols, acids or fats. The use of plants or plant extracts in cosmetics is rampant. There are a great many of different uses, such as humidification, brighteners, tanning lotions, make-ups, sun filters, scavengers, antioxidants, immunity stimulation, detergents, preserving agents or thickening agents.

However there are restrictions with respect to the use of plants or plant components. i.e.: The availability may be restricted, e.g. by the seasons, limited storage capacities, protection of species, problems in cultivation, or bad harvests. The quality is not unchanging, e.g. due to seasonal variations, different cultivation methods, geographic differences, different suppliers, clones, pollution of the environment, or physical status.

Plant cell culture techniques have been shown to help mitigate these drawbacks (US2014072619A1, EP1244464, FR2837385, US2006021084, WO2005108596, WO2005070066, among others).

When certain known process steps are followed, it is possible to obtain uniform dedifferentiated cells (herein identified as meristematic cells, or simply stem cells), showing the following advantages as compared with cultivated whole plants: Independency from seasons; Continuous production including freedom from pollution of environment and other impurities; manageable and reproducible production of metabolites; protection of rare or limited plant reserves; and no limitation of market availability.

Morinda citrifolia, M. citrifolia or Noni, from the Indian Mulberry plant, is known to effectively boost hydration and firmness of the surface layer of the skin that stimulates the body's natural production of collagen. M. citrifolia restores visible tone and elasticity to the surface of the skin, and minimizes the apparent effects of environmental aggressions such as pollution, sun, and stress. M. citrifolia is high in antioxidants that help fight free-radical damage caused by the sun and other elements. M. citrifolia is also rich in linoleic acid, which is an essential fatty acid with specific abilities for nourishing the health of the skin. Each of these, along with other ingredients, aids in barrier repair and protection, or repair and protection of the stratum corneum. Thus, M. citrifolia is found in dermatological formulations. US2002192246 and US2002187168 disclose and claim an intensive repair serum and cosmetic skin care toner, respectively, for skin treatment comprising Morinda citrifolia fruit juice. WO2005030141 and WO2005086937 disclose compositions comprising M. citrifolia for oral care or oral administration, respectively.

Gossypium herbaceum, G. herbaceum, is commonly known as Levant cotton and is a species of cotton native to the semi-arid regions of sub-Saharan Africa and Arabia. The cotton plant itself has medicinal uses, and can be cultivated traditionally, in house backyards, for, e.g., women's menstrual cycle pains and irregular bleeding. It is also known to be used after birth to expel the placenta and to increase the lactation, as well as for gastrointestinal issues, such as hemorrhages and diarrhea, for nausea, fevers and headaches.

The cited prior art documents disclose the use of certain parts or of extracts of certain parts of plants for use in compositions for human care. ES2579385B1 discloses a different approach by using plant cell cultures of G. herbaceum, wherein isolated plant cells are submitted to cell dedifferentiation until totipotency is reached and then submitted to a rupture process for lysis of the cells. US2014072619 discloses the use of cell lysates of the plant Malus domestica in cosmetic and pharmaceutic compositions. Cell lysates allow tapping into the complex matrix of constituents of dedifferentiated plant cells, which include among others salts, acids, polyphenols, sugars, fats and proteins.

A method for obtaining plant cell lysates can be found in ES2579385B1.

### SUMMARY OF THE INVENTION

The prior art does not disclose combinations of M. citrifolia with G. herbaceum. Nonetheless, the inventors have surprisingly found that a particular combination of cell lysates of M. citrifolia and cell lysates of G. herbaceum has a synergistic effect in reducing reactive oxygen species in skin cells.

Accordingly, in a first aspect, the invention is directed to a composition comprising:
- a Morinda citrifolia dedifferentiated cell lysate at a concentration equal to or higher than 0.001% and lower than 1%, by weight ratio relative to the total weight of the composition;
- a Gossypium herbaceum dedifferentiated cell lysate at a concentration equal to or higher than 0.001% and lower than 1%, by weight ratio relative to the total weight of the composition.

The compositions of the invention can be used for application to healthy skin that suffers from oxidative stress. For example, skin that displays signs of ageing, cutaneous discomfort or cutaneous dryness. In other specific cases, the compositions of the invention can be applied to the skin when it already exhibits clinical signs of a pathological deficiency in the cutaneous barrier.

Therefore, the compositions of the invention are of use as cosmetic and pharmaceutical compositions, particularly for skin conditions associated with inflammatory episodes.

Therefore, in a second aspect, the invention is directed at a cosmetic or pharmaceutical composition comprising the composition of the first aspect, further comprising a cosmetically or pharmaceutically acceptable excipient.

In a third aspect, the invention is directed at a pharmaceutical skin composition of the invention, for use in the prevention and/or treatment of dermic inflammatory conditions.

In a fourth aspect, the invention is directed at a non-therapeutic use of the cosmetic composition of the invention to densify dermis, strengthen dermal-epidermal junction, thicken epidermis and/or provide anti-glycation defense means to the skin, or for the non-therapeutic amelioration or the non-therapeutic counteraction of a skin condition selected from dry skin, roughening skin, aging skin, and skin wrinkle.

### DETAILED DESCRIPTION OF THE INVENTION

Unless defined otherwise, all technical and scientific terms used herein have the same meaning as commonly understood to one of ordinary skill in the art to which this disclosure belongs.

### Definitions

The open term "comprising" is to be understood as a generalization of the closed term "consisting". Therefore, the skilled person readily understands that the term "consisting" is contemplated within the meaning of "comprising".

The term "topical" refers to the administration of a compound by applying it on the body surface and includes, but is not limited to, dermal administration and administration through the mucosa.

The term "lysate" is to be interpreted as the material obtained upon the destruction or the dissolution of plant cells (lysis). Said material comprises the intracellular biological constituents naturally contained inside the cells. For the purposes of the present invention, the term "lysate" is used to denote the whole lysate obtained via lysis of the plant cells. The lysate is thus characterized by comprising the intracellular biological constituents, the released molecules to the extracellular medium and the constituents of the cell walls and membranes. In a particular embodiment, the lysate also comprises molecules originating from the growth culture of the cells. Non-limiting examples of such molecules are those originated from the plant growth medium, including the Murashige and Skoog medium or the Hoagland solution, auxins, cytokins and methyl jasmonate.

"Meristematic cells" are uniform undifferentiated (or dedifferentiated) cells having the property of constant regeneration and the unique ability of turning into any other cell type by cleavage and differentiation. They can also be identified as stem cells (from plant material only).

The term "biomass" corresponds to the mass present in the composition of the invention, which is of plant origin.

The term "dry residue", corresponds to the non-volatile solids present in the lysates.

The term "excipient" refers to a vehicle, diluent or adjuvant suitable for dermal administration, preferably topical administration, which is non-toxic and which does not show harmful interaction with any components of the composition.

A "cosmetically and/or pharmaceutically acceptable excipient" is a compound suitable for cosmetic and/or pharmaceutical use, which is physiologically tolerable and does not normally cause an allergic reaction, or similar adverse reactions, when administered to animals, particularly human beings.

When mass values are provided in terms of percentage, unless stated otherwise, they are to be interpreted as mass percentages.

Unless stated otherwise, when ranges are provided, the numbers defining the lower and upper limits are included in the range.

### Skin impairment

Impairment of skin barrier function is often accompanied by an altered integrity of the stratum corneum and a consequential increase in transepidermal water loss. Thus, impaired barrier function and skin dryness are intimately inter-related.

A skin barrier disorder may be generally defined as a state in which the skin, or more particularly the epidermis and especially the horny layer, does not exhibit a normal barrier function. This deterioration of the skin may merely be a minor aesthetic alteration and/or a sensation of cutaneous discomfort or cutaneous dryness in a skin considered as healthy but also a pathological condition. Specific examples of skin barrier disorders are dry skin, atopic dermatitis, contact dermatitis and the like. Further examples contemplated herein are described below and defined in the claims.

Dry skin, also called xerosis, is a very common skin abnormality. A dry skin has a rough feel, appears covered with squamae and manifests itself essentially through a sensation of tautness and/or tension.

Dry skin is in fact accompanied by a desquamation disorder and presents various stages as a function of the severity of this desquamation. When the skin is slightly dry, these squamae are abundant but sparingly visible to the naked eye, and removal takes place corneocyte by corneocyte. They are increasingly infrequent but increasingly visible to the naked eye when this disorder worsens, and the patches may comprise several hundred corneocytes, thus representing more or less large patches, known as squamae.

A skin suffering from skin dryness generally displays the following signs, namely a rough and flaky feel, and also decreased suppleness and elasticity. There are also studies that have demonstrated an incidence of dryness on the origin and formation of wrinkles and fine lines, and indeed, in visual terms a dry skin makes said wrinkles and fine lines more apparent.

Moreover, from a sensory viewpoint, skin dryness is characterized by a sensation of tautness and/or itching. For obvious reasons, these manifestations are not only sources of discomfort, or even pain, but also an unaesthetic appearance.

The origin of this dryness may be of constitutional or acquired type. In the case of acquired dry skin, the involvement of external parameters such as exposure to chemical agents, to harsh climatic conditions, to sunlight or else to certain therapeutic treatments (for example retinoids) is a determining factor. Under these external influences, the epidermis may then become momentarily and locally dry. This may concern any type of epidermis.

Indeed, skin is known to have tendency to drying due to environmental, psychological and hormonal factors. In particular, pollution is an increasing concern worldwide due to the industrialization and the release of high quantity of chemical compounds in the air. These toxic components adhere to the skin's surface and disturb the horny layer's integrity. This leads to many damages such as the loss of hydration. Climatic conditions such as cold or wind are also known to promote skin dryness.

In view of the increasing dangerous environmental factors, it is important nowadays that skin be well moisturized and does not suffer a water loss that would lead to skin wilting and drying. Therefore, treating and preventing cutaneous dryness has become essential, not only from a therapeutic viewpoint, but also from an aesthetic viewpoint.

Atopic dermatitis (AD), also referred to as atopic eczema, is a chronic disease of the skin in which the skin is dry, easily irritated, allergen predisposed, typically scaly, often thickened, commonly red, sometimes exudative, frequently infected and above all itchy. In industrialized countries, the prevalence rates in childhood range from 15 to 30 percent and in adults from 2 to 10 percent of the general population. Atopy appears to show a strong hereditary component, but as for other skin barrier disorders, environmental aggressions are also thought to play an important role in the development of atopy.

The inventors of the present invention have found that lysates of Morinda citrifolia and Gossypium herbaceum can be favorably used for enhancing the human skin barrier function at both cosmetic and pharmaceutical levels.

### Lysates

In a particular embodiment, the lysates of the invention have activity on skin cells as, at least one of the following: antioxidant, anti-inflammatory, anti-proliferative, anti-pruritic, anti-degranulation and as moisturizer.

Accordingly, in a first aspect, the invention is directed to a composition comprising:
- a Morinda citrifolia dedifferentiated cell lysate at a concentration equal to or higher than 0.001 % and lower than 1%, by weight ratio relative to the total weight of the composition;
- a Gossypium herbaceum dedifferentiated cell lysate at a concentration equal to or higher than 0.001% and lower than 1%, by weight ratio relative to the total weight of the composition.

In a particular embodiment, the invention is directed to a composition comprising a Morinda citrifolia dedifferentiated cell lysate at a concentration equal to or higher than 0.001%, 0.002%, 0.003%, 0.005%, 0.01%, 0.03%, 0.05%, 0.09%, 0.1%, 0.3% or 0.5%, by weight ratio relative to the total weight of the composition.

In another particular embodiment, the invention is directed to a composition comprising a Morinda citrifolia dedifferentiated cell lysate at a concentration lower than 1%, 0.95%, 0.9%, 0.85%, 0.80%, 0.75%, 0.7%, 0.65%, 0.6%, 0.55% or 0.5%, by weight ratio relative to the total weight of the composition.

In a particular embodiment, the invention is directed to a composition comprising a Gossypium herbaceum dedifferentiated cell lysate at a concentration equal to or higher than 0.001%, 0.002%, 0.003%, 0.005%, 0.01%, 0.03%, 0.05%, 0.09%, 0.1%, 0.3% or 0.5%, by weight ratio relative to the total weight of the composition.

In another particular embodiment, the invention is directed to a composition comprising a Gossypium herbaceum dedifferentiated cell lysate at a concentration lower than 1%, 0.95%, 0.9%, 0.85%, 0.80%, 0.75%, 0.7%, 0.65%, 0.6%, 0.55% or 0.5%, by weight ratio relative to the total weight of the composition.

In another particular embodiment, the invention is directed to a composition comprising a Morinda citrifolia dedifferentiated cell lysate at a concentration comprised between 0.001% and 0.9% by weight ratio relative to the total weight of the composition, preferably comprised between 0.01% and 0.9%, more preferably comprised between 0.05% and 0.8%, even more preferably comprised between 0.09% and 0.6%.

In yet another particular embodiment, the invention is directed to a composition comprising a Gossypium herbaceum dedifferentiated cell lysate at a concentration comprised between 0.001% and 0.9% by weight ratio relative to the total weight of the composition, preferably comprised between 0.01% and 0.9%, more preferably comprised between 0.05% and 0.8%, even more preferably comprised between 0.09% and 0.6%.

The lysis may be accomplished via various techniques, such as an osmotic shock, a heat shock, via ultrasonic or centrifugation. More particularly, the lysate may be obtained according to the disclosures found in US4464362 and EP43128. The preferred method for obtaining the cell lysates is the one disclosed in ES2579385B1.

The method of ES2579385B1 can be reproduced for producing the cell lysates of the invention.

In a particular embodiment, the G. herbaceum lysates are obtainable by following the steps:
a) Growing the G. herbaceum plant cells in Murashige & Skoog (MS) medium comprising plant hormones (preferably 2 mg/L of 2,4-dichlorophenoxyacetic acid) and/or cytokines (preferably 1 mg/L of kinetin) for 7 days under stirring (90-115 rpm), in the dark and at a temperature of 20-25 °C;
b) Adding a composition comprising cyclodextrins (such as the ones sold by Wacker, ref. 121877) at a final concentration of 36 g/L and sucrose at a final concentration of 30 g/L;
c) Adding a first composition comprising methyl jasmonate at a final concentration of 150 µM;
d) Stirring for 3 days the composition resulting from step c) at 90-115 rpm, in the dark and at a temperature of 20-25 °C;
e) Adding a second composition comprising methyl jasmonate at a concentration of 150 µM and repeating the conditions of step d) for 2 days;
f) Adding a third composition comprising methyl jasmonate so that the final concentration of methyl jasmonate is 150 µM and repeating the conditions of step d) for 2 additional days;
g) Lysing the cells of the composition obtained in the previous step in an Ultraturrax T-25 Basic apparatus at a temperature below 30 °C, using a dispersing tool (IKA S 25 N - 25 F). The lysis progress can be followed by optic microscopy.

At step f), the polyphenol and flavonoid concentration can be quantified following the procedure of Marinova O et al., "TOTAL PHENOLICS ANO TOTAL FLAVONOIOS IN BULGARIAN FRUITS ANO VEGETABLES", Journal of University of Chemical Technology and Metallurgy, 2005, Vol. No. 40 (3), pp.: 255-260.

In a preferred embodiment, the lysate obtained from the steps above has a biomass concentration of 0.36 g/mL, preferably 0.363 g/mL.

The skilled person readily understands that the method above is not intended to limit the invention in any way, and merely serves as a means to produce the lysates of the invention.

In this sense, it is also clear to the skilled person that if the lysates are prepared by any other equivalent means (such as different growth medium, different periods of time for cell growth, different temperatures, different concentrations of eliciting compounds, to name a few), the resulting lysates can also be used in the present compositions. The only care that should be taken is that the lysate weight percentage values are adapted so that the dry weight of the cell residue before lysis, the density, the cell number and/or the biomass concentration are within the ranges disclosed herein and defined in the claims.

In a particular embodiment of the method for obtaining the lysate of G. herbaceum, an optional before the lysis step may be performed. This optional step comprises adding glycerin and citric acid monohydrate so that the final concentrations after the lysis step are 68.5% glycerin, 30% cell lysate and 1,5% citric acid monohydrate. The steps described above provide a 30% lysate of dedifferentiated G. herbaceum cells in glycerin, at a density of 1.10-1.30 g/mL, preferably 1.21 g/mL. The lysate obtained from the steps above has a biomass concentration of 0.363 g/mL.

The method of ES2579385B1 can also be reproduced for producing the cell lysates of the M. citrifolia.

In a particular embodiment, the M. citrifolia lysates are obtainable by following the steps:
a) Growing the M. citrifolia plant cells in Murashige & Skoog (MS) medium comprising plant hormones (preferably 2 mg/L of naphthaleneacetic acid) and/or cytokines (preferably 0.2 mg/L of kinetin) for 14 days under stirring (110-120 rpm), in the dark and at a temperature of 20-25 °C;
b) Cell suspension in a) is diluted one time with Murashige & Skoog (MS) medium comprising plant hormones (preferably 0.5 mg/L of naphthaleneacetic acid) and/or cytokines (preferably 0.5 mg/L of kinetin) with sucrose at a final concentration of 80g/L;
c) Adding a first composition comprising cyclodextrins (such as the ones sold by Wacker, ref. 121877) at a final concentration of 72 g/L;
d) Adding a first composition comprising methyl jasmonate at a final concentration of 500 µM;
e) Stirring for 14 days the composition resulting from step d) at 110-120 rpm, in the dark and at a temperature of 20-25 °C;
f) Lysing the cells of the composition obtained in the previous step in an Ultraturrax T-25 Basic apparatus at a temperature below 30 °C, using a dispersing tool (IKA S 25 N - 25 F). The lysis progress can be followed by optic microscopy.

At step c), the terpenoids concentration can be quantified following the procedure of Narayan G, et al. Estimation of total Terpenoids concentration in plant tissues using a monoterpene, Linalool as standard reagent. 27 November 2012, PROTOCOL (Version 1) available at Protocol Exchange [+https://doi.org/10.1038/protex.2012.055+]

In a preferred embodiment, the lysate obtained from the steps above has a biomass concentration of 0.28 g/mL, preferably 0.280 g/mL.

The skilled person readily understands that the method above is not intended to limit the invention in any way, and merely serves as a means to produce the lysates of the invention.

In this sense, it is also clear to the skilled person that if the lysates are prepared by any other equivalent means (such as different growth medium, different periods of time for cell growth, different temperatures, different concentrations of eliciting compounds, to name a few), the resulting lysates can also be used in the present compositions. The only care that should be taken is that the lysate weight percentage values are adapted so that the dry weight of the cell residue before lysis, the density, the cell number and/or the biomass concentration are within the ranges disclosed herein and defined in the claims.

In a particular embodiment of the method for obtaining the lysate of M. citrifolia, the optional step before the lysis may be performed. This optional step comprises adding glycerin and the citric acid monohydrate, so that the final concentrations after the lysis step are 68,5% glycerin, 30% cellular composition from step e) and 1,5% citric acid monohydrate. The steps described above provide a 30% lysate of dedifferentiated M. citrifolia cells in glycerin, at a density of 1.20 g/mL (ranging from 1.10-1.30 g/mL). The lysate obtained from the steps above has a biomass concentration of 0.280 g/mL.

In a preferred embodiment, the composition of the invention is a composition wherein:
- said Morinda citrifolia dedifferentiated cell lysate is a lysate characterized by a biomass concentration of 0.28 g/mL; and/or
- said Gossypium herbaceum dedifferentiated cell lysate is a lysate characterized by a biomass concentration of 0.36 g/mL.

In a particular embodiment, 1 L of the cell culture of G. herbaceum used before lysis has a dry residue of at least 40 g, at least 50 g, at least 60 g and at least 70 g, at least 80 g and at least 90 g. In a preferred embodiment, 1 L of the cell culture of G. herbaceum used before lysis has a dry residue of at least 60 g.

In another particular embodiment, 1 L of the cell culture of M. citrifolia used before lysis has a dry residue of at least 40 g, at least 50 g, at least 60 g, at least 70 g, at least 80 g and at least 90 g. In a preferred embodiment, 1 L of the cell culture of M. citrifolia used before lysis has a dry residue of at least 80 g.

In another particular embodiment, the composition of the invention comprises:
- a Morinda citrifolia dedifferentiated cell lysate at a concentration equal to or higher than 0.001% and lower than 1%, by weight ratio relative to the total weight of the composition, wherein 1L of said Morinda citrifolia lysate has a dry residue of at least 80 g; and/or
- a Gossypium herbaceum dedifferentiated cell lysate at a concentration equal to or higher than 0.001% and lower than 1%, by weight ratio relative to the total weight of the composition, wherein 1L of said Gossypium herbaceum lysate has a dry residue of at least 60 g.

In a particular embodiment, the composition of the invention comprises:
- from 0.00028% to less than 0.28% of biomass resulting from Morinda citrifolia dedifferentiated cell lysate, by weight ratio relative to the total weight of the composition; and/or
- from 0.00036% to less than 0.36% of a dry residue of Gossypium herbaceum dedifferentiated cell lysate, by weight ratio relative to the total weight of the composition.

In a particular embodiment, the dedifferentiated plant cell culture is cultured so that the cell density, expressed as number of cells per volume unit of suspension culture, ranges from 1×10⁵ cells/mL of cell culture to 1×10⁷ cells/mL cell culture.

In a particular embodiment, the density of the G. herbaceum lysate or of the M. citrifolia lysate is comprised between 1.10 and 1.30 g/mL, preferably between 1.20 and 1.25 g/mL.

In a particular embodiment, the lysate of M. citrifolia, or the lysate of G. herbaceum, is a dedifferentiated cell lysate comprising from 10% to 50%, preferably from 20% to 40%, even more preferably from 25% to 35% by weight of plant-derived active materials.

The lysates may further comprise proteins, lipids, phytosterols, sugars and polyphenols.

In a particular embodiment, the lysates of M. citrifolia comprise biomolecules selected from polyphenols, phytosterols, glucose, fructose, saccharose, furanose, arabitol, myo-inositol, lipids and proteins. In another particular embodiment, the lysates of G. herbaceum comprise biomolecules selected from polyphenols, phytosterols, glucose, fructose, saccharose, furanose, arabitol, myo-inositol, lipids and proteins.

In a particular embodiment, the lysates comprise polyphenols in a concentration ranging from 100 to 700 mg/L of lysate. Preferably, from 250 to 500 mg/L, even more preferably from 300 to 400 mg/L of lysate. More preferably, the latter lysates are G. herbaceum lysates.

In another particular embodiment, the lysates comprise phytosterols in a concentration ranging from 10 to 80 mg/L of lysate. Preferably, from 30 to 60 mg/L, even more preferably from 40 to 50 mg/L of lysate. More preferably, the latter lysates are G. herbaceum lysates.

In particular, the lysates may comprise a minimum of 90 mg/L of proteins, 30-60 mg/L of phytosterols (β-sitosterol, stigmasterol, lanosterol), triglycerides, fatty acids, stearic acid, monopalmitin, monostearin, sugars (glucose, fructose, sucrose, furanose, 2-keto-D-gluconic acid, 6-deoxy-β-L-mannofuranose, α-D-glucopyranose, arabitol, myo-inositol) and 250-500 mg/L of polyphenols. More preferably, the latter lysates are G. herbaceum lysates.

In a particular embodiment, 1 L of each of the lysates, preferably of G. herbaceum, comprise at least 1000, at least 1500, preferably at least 2000 mg of soluble sugars.

In another particular embodiment, the lysates have a pH value comprised between 1.0 and 6, preferably between 1.5 and 5.5, more preferably between 2.0 and 4.5, even more preferably between 2.0 and 3.5.

### Excipients

The cosmetic or pharmaceutical compositions of the invention may include, in addition to the lysates according to the present invention, which are the active ingredients, a cosmetically or pharmaceutically acceptable excipient selected from a vehicle, diluent or an adjuvant.

Therefore, in a particular embodiment, the composition of the invention comprises at least one cosmetically and/or pharmaceutically acceptable excipient. Preferably, the excipient is selected from a vehicle, diluent or adjuvant.

In a particular embodiment, the composition of the invention comprises at least one cosmetically or pharmaceutically acceptable excipient. In this case, the excipient is found in an amount from 5% to 70%, from 10% to 70%, from 15% to 70%, preferably from 15% to 60%, more preferably from 20% to 60%, even more preferably from 20% to 55% by weight of the composition.

The selection of suitable excipients is a routine task for the person skilled in the art depending on the type of product in which the composition is to be formulated. In the CTFA Cosmetic Ingredient Handbook, 7a Ed, 1997, and also in the 8^{a} Ed, 2000, a wide variety of cosmetically and/or pharmaceutically acceptable excipients commonly used in skin care compositions which can be incorporated in the compositions of the present invention are described. Some non-limiting examples of suitable vehicles, diluents and adjuvants include an aqueous or organic solvent, a thickener (ionic or nonionic) or a gelling agent or moisturizing agent, a softener, an antioxidant or preservative or stabilizer including a vitamin, an opacifier or colorant or pigment, an emollient including fat bodies and further moisturizing agents, an anti-foaming agent, a perfume or fragrance agent, a surfactant or emulsifier; a sequestrant, an alkalizing agent, an acidifier agent, a sun screen agent, and mixtures thereof.

The function of some excipients may overlap. This is readily understood by the skilled person and is known to occur. For example, a moisturizing agent can be both a gelling agent, a thickener and an emollient. In turn, an emollient can also function as a surfactant, for example.

Preferred excipients are described below.

A suitable vehicle includes any such material known in the art such as, for example, any sterile liquids, such as water and oils, including those of petroleum, animal, vegetable or synthetic origin, such as peanut oil, soybean oil, mineral oil, sesame oil and similar, gel, solvent, liquid diluent, solubilizer, or the like, which is non-toxic and which does not show harmful interaction with any components of the composition.

In a preferred embodiment, the vehicle is selected from water, glycerin, propylene glycol, polyethylene glycol, polypropylene glycol, sorbitol esters, 1,2,6-hexanetriol, ethanol, isopropanol, diethyl tartrate, butanediol, and mixtures thereof. In a preferred embodiment, the composition of the invention comprises water and glycerin.

In a particular embodiment, the composition comprises from 25% to 90%, preferably from 28% to 88%, more preferably from 35% to 85%, even more preferably from 40% to 80% of the vehicle. Preferably, said vehicle is at least water. In a most preferred embodiment, the balance of the composition is water.

Additives to the aqueous phase are, inter alia agents that prevent the compositions from drying out, for example humectants, such as polyalcohols, such as glycerol, sorbitol, propylene glycol and/or polyethylene glycols.

In a particular embodiment, the composition of the invention further comprises glycerin. In a particular embodiment, the composition comprises from 1% to 15%, preferably from 1% to 10%, more preferably from 1% to 8%, even more preferably from 1% to 7% of glycerin.

More preferably, the composition of the invention comprises:
- from 35% to 85% of water, preferably from 40% to 80%; and
- from 1% to 8% of glycerin, preferably from 1% to 7%.

Suitable emulsifiers or surfactants are surface-active substances having predominantly hydrophilic properties, for example C18-C26 alkyl phosphates, and fatty acid esters of polyalcohols and/or ethylene oxide adducts thereof, including C8-18 fatty acid esters of (poly)ethylene glycol, (poly)propylene glycol or sorbitol, polyglycerol fatty acid esters or polyoxyethylene sorbitan fatty acid esters (Tweens). Further exemplary compounds are C8-C12 fatty acid glycerides such as caprylic/capric mono- di- or tri-glycerides, and polysorbates including Polysorbate 20 (Tween 20) and polysorbate 80 (Tween 80).

In the compositions of the present invention, emulsifiers or surfactants may be present from 0.1% to 30%, preferably between 0.5% and 20% by weight of the total composition.

Due to their versatile nature, emulsifier or surfactant compounds may also function as emollients. Emollients prevent evaporation of water from the skin and, in general, the more lipid in the formulation, the greater the emollient effect.

Emollients may further be incorporated into the compositions of the present invention. Levels of such emollients may range from 0.5% to 70%, preferably between 5% and 60% by weight of the total composition.

Emollients may be classified as esters, fatty acids and alcohols, polyols and hydrocarbons.

Suitable emollients include petrolatum (white soft paraffin), castor oil, cetyl alcohol, cetearyl alcohol, cocoa butter, isopropyl myristate, isopropyl palmitate, lanolin, liquid paraffin, polyethylene glycols, shea butter, silicone oils, stearic acid, stearyl alcohol, and mineral oils. Oily bases that can be used are fatty alcohols, especially those containing from 12 to 18 carbon atoms, for example lauryl, cetyl or stearyl alcohol, fatty acids, especially those containing from 10 to 18 carbon atoms, for example palmitic or stearic acid, fatty acid esters, e.g. glyceryl tricaprilocaprate (neutral oil) or cetyl palmitate, liquid to solid waxes, for example isopropyl myristate, wool wax or beeswax, and/or hydrocarbons, especially liquid, semi-solid or solid substances or mixtures thereof, for example petroleum jelly (petrolatum, Vaseline) or paraffin oil.

Examples of emollients are:
I. Hydrocarbon waxes and oils such as mineral oils, petrolatum, paraffin, isoparaffins, ceresin, microcrystalline wax, polyethylene, squalene and perhydrosqualene.
II. Silicone oils such as dimethylpolysiloxanes, methylphenylpolysiloxanes and water-soluble and alcohol-soluble glycol-silicone copolymers.
III. Triglycerides, such as animal and vegetable fats and oils. Examples include, but are not limited to, castor oil, cod liver oil, corn oil, olive oil, almond oil, palm oil, sesame oil, cotton seed oil and soybean oil.
IV. Acetoglyceride esters, such as acetylated monoglycerides.
V. Ethoxylated glycerides, such as ethoxylated glycerol monostearate.
VI. Alkyl esters of fatty acids, preferably having 10 to 20 carbon atoms. Methyl, isopropyl and butyl esters of fatty acids are useful herein. Examples include, but are not limited to, hexyl laurate, isohexyl laurate, isohexyl palmitate, isopropyl palmitate, decyl oleate, isodecyl oleate, hexadecyl stearate, decyl stearate, isopropyl isostearate, diisopropyl adipate, dibutyl adipate, diisohexyl adipate, dihexyldecyl adipate, diethyl sebacate, diisopropyl sebacate, diisopropyl dimerate, lauryl lactate, dioctyl succinate, myristoyl lactate and cetyl lactate. Further examples include triisopropyl trilinoleate and trilauryl citrate, lauryl palmitate, myristyl lactate, stearyl oleate, propylene glycol myristyl ether acetate, isononyl isononanoate and cetyl octanoate.
VII. Alkenyl esters of fatty acids having 10 to 20 carbon atoms. Examples thereof include, but are not limited to, oleyl myristate, oleyl stearate and oleyl oleate.
VIII. Fatty acids having 4 to 26, preferably 8 to 24, more preferably 10 to 20 carbon atoms. Suitable examples include, but are not limited to, pelargonic, lauric, myristic, palmitic, stearic, isostearic, hydroxystearic, oleic, linoleic, ricinoleic, arachidonic, behenic and erucic acids. Other appropriate examples include the fatty acids derivatives of 1-octanol (C8), 1-Nonanol (C9), 1-Decanol (C10), Undecanol (C11), Dodecanol (C12), myristyl (C14), cetyl or palmitic (C16), stearyl (C18), oleyl (C18), linoleyl (C18), arachidyl (C20), behenyl (C22) or lignoceryl (C24).
IX. Fatty alcohols having 4 to 26, preferably 8 to 24, more preferably 10 to 20 carbon atoms. Lauryl, myristoyl, palmitoyl, stearyl, isostearyl, hydroxystearyl, oleyl, ricinoleyl, behenyl, erucyl and 2-octyl dodecanol alcohols are appropriate examples of fatty alcohols. Other appropriate examples include 1-octanol (C8), 1-Nonanol (C9), 1-Decanol (C10), Undecanol (C11), Dodecanol (C12), myristyl (C14), cetyl or palmitic (C16), stearyl (C18), oleyl (C18), linoleyl (C18), arachidyl (C20), behenyl (C22) or lignoceryl (C24) alcohols.
X. Fatty alcohol ethers. Ethoxylated fatty alcohols having 10 to 20 carbon atoms include, but are not limited to, lauryl, cetyl, stearyl, isostearyl, oleyl and cholesterol alcohols having attached thereto from 1 to 50 ethylene oxide groups or 1 to 50 propylene oxide groups.
XI. Ether-esters, such as fatty acid esters of ethoxylated fatty alcohols.
XII. Lanolin and derivatives. Lanolin, lanolin oil, lanolin wax, lanolin alcohols, lanolin fatty acids, isopropyl lanolate, ethoxylated lanolin, ethoxylated lanolin alcohols, ethoxylated cholesterol, propoxylated lanolin alcohols, acetylated lanolin, acetylated lanolin alcohols, lanolin alcohols linoleates, lanolin alcohols ricinoleate, acetate of lanolin alcohols ricinoleate, hydrogenolysis of lanolin, and liquid or semisolid lanolin absorption bases are illustrative examples of lanolin derived emollients.
XIII. Polyhydric alcohols and polyether derivatives. Propylene glycol, dipropylene glycol, polypropylene glycol 2000 and 4000, polyoxyethylene polypropylene glycols, glycerol, ethoxylated glycerol, propoxylated glycerol, sorbitol, ethoxylated sorbitol, hydroxypropyl sorbitol, polyethylene glycol 200-6000, methoxy polyethylene glycols 350, 550, 750, 2000, 5000, poly(ethylene oxide) homopolymers (100,000-5,000,000), polyalkylene glycols and derivatives, hexylene glycol (2-methyl-2,4-pentanediol), 1,3-butylene glycol, 1,2-hexanediol, 1,2,6-hexanetriol, ethohexadiol USP (2-ethyl-1,3-hexanediol), and polyoxypropylene derivatives of trimethylolpropane are suitable examples.
XIV. Polyhydric alcohol esters. Mono- and di-acyl esters of ethylene glycol, mono- and di-acyl esters of diethylene glycol, mono- and di-acyl esters of polyethylene glycol (200-6000), mono- and di-acyl esters of propylene glycol, polypropylene glycol 2000 monooleate, polypropylene glycol 2000 monostearate, ethoxylated propylene glycol monostearate, mono- and di-acyl esters of glycerol, poly-acyl esters of poly glycerol, glycerol monostearate, 1,3-butylene glycol monostearate, 1,3-butylene glycol distearate, isostearyl palmitate and isostearyl stearate, acyl ester of polyoxyethylene polyol, acyl esters of sorbitan, 2-ethyl-hexyl myristate and acyl esters of polyoxyethylene sorbitan are suitable examples.
XV. Waxes such as beeswax, spermaceti, myristoyl myristate and stearyl stearate.
XVI. Beeswax derivatives, such as polyoxyethylene sorbitol beeswax. These are reaction products of beeswax with ethoxylated sorbitol of varying ethylene oxide content that form a mixture of ether-esters.
XVII. Vegetable waxes, including, but not limited to, carnauba and candelilla waxes.
XVIII. Phospholipids such as lecithin and derivatives.
XIX. Sterols. Examples include, but are not limited to, cholesterol and acyl esters of cholesterol.
XX. Amides, such as fatty acid amides, ethoxylated acyl amides and solid fatty acid alkanolamides.

Some emollients previously described also have emulsifying properties. When a lotion contains one of these emollients, an additional emulsifier is not needed, though it can optionally be further included in the formulation.

The compositions of the present invention may further comprise thickeners or gelling agents. A thickener or gelling agent (or even moisturizing agent) will usually be present in amounts anywhere from 0.1 to 20% by weight, preferably from about 0.5% to 10% by weight of the composition. Exemplary excipients are cross-linked polyacrylate materials available under the trademark Carbopol from the B.F. Goodrich Company. Gums may be employed such as xanthan, carrageenan, gelatin, karaya, pectin and locust beans gum. Further examples include hyaluronate crosspolymers. Under certain circumstances the thickening, gelling or moisturizing function may be accomplished by a material also serving as a silicone or emollient. For instance, silicone gums and esters such as glycerol stearate have dual functionality.

In a particular embodiment, the composition of the present invention may also include sun screen agents. Sun screen agents include compounds commonly employed to block ultraviolet light and are described in detail in "Sunscreens: Development, Evaluation and Regulatory Aspects" N. Shaath et al., 2a Ed, pages 269-273, Marcel Dekker, Inc. (1997) and "Sun Protection Effect of Nonorganic Materials" S. Nakada & H. Konishi, Fragrance Journal, volume 15, pages 64-70 (1987).

Illustrative sun screen agents are the derivatives of PABA, cinnamate and salicylate. In a particular embodiment, the composition of the invention comprises a sun screen agent selected from Avobenzone (Butyl Methoxydibenzoylmethane, Parsol 1789), Bisdisulizole disodium (Neo Heliopan AP), Diethylamino hydroxybenzoyl hexyl benzoate (Uvinul A Plus), Ecamsule (Mexoryl SX), Menthyl anthranilate, Amiloxate, 4-Aminobenzoic acid (PABA), Cinoxate, Ethylhexyl triazone (Uvinul T 150), Homosalate, 4-Methylbenzylidene camphor (Enzacamene), Octyl methoxycinnamate (Octinoxate), Octyl salicylate (Octisalate), Padimate O (Escalol 507), Phenylbenzimidazole sulfonic acid (Ensulizole), Polysilicone-15 (Parsol SLX), Trolamine salicylate, Bemotrizinol (bis-ethylhexyloxyphenol methoxyphenyl triazine, Tinosorb S), benzophenone derivatives including Mexenone, Dioxybenzone, Oxybenzone (Eusolex 4360) or Sulisobenzone, Drometrizole trisiloxane (Mexoryl XL), Iscotrizinol (Uvasorb HEB), Octocrylene, Bisoctrizole (Tinosorb M), Titanium dioxide and Zinc oxide. The exact amount of sun screen employed in the emulsions can vary depending upon the degree of protection desired from the sun's UV radiation.

If present, each of the sun screen agents included in the composition of the invention is in an amount between 0.1% and 10%, between 0.2% and 8%, and preferably between 0.5% and 8% by weight of the composition.

The composition may further comprise preservative or antioxidant agents. Exemplary preservative compounds that can be used in the compositions of the invention are selected from butylated hydroxytoluene (BHT), hydroquinone, tocopherol and derivatives thereof, as well as flavonoids, or miscellaneous antioxidants like ascorbic acid, vitamin E, ascorbyl palmitate or Ascorbyl stearate. Typical amounts range from 0.01% and 10%, between 0.05% and 8%, and preferably between 0.1% and 5% by weight of the composition.

In another embodiment, the composition further comprises acids or bases as pH buffer excipients, such as isocitric acid, citric acid, aconitic acid, and propane-1,2,3-tricarboxylic acid.

Other adjunct minor components may also be incorporated into the compositions of the invention. These ingredients may include opacifiers, colorants or pigments and perfume or fragrance agents. Amounts of these other adjunct minor components may range anywhere from 0.001% up to 20% by weight of the composition.

Powders may be incorporated into the cosmetic composition of the invention.

These powders include chalk, talc, kaolin, starch, smectite clays, chemically modified magnesium aluminum silicate, organically modified montmorillonite clay, hydrated aluminum silicate, fumed silica, aluminum starch octenyl succinate and mixtures thereof.

The weight ratios described herein are percentages relative to the total weight of the composition. Therefore, the total weight of the composition is 100%. If a combination of excipients is described in terms of their weight ratio ranges, the sum of their weight cannot exceed 100% of total weight, including the lysates.

### The compositions

In a second aspect, the invention is directed at a cosmetic or pharmaceutical composition comprising the composition of the first aspect, further comprising a cosmetically or pharmaceutically acceptable excipient.

The cosmetic or pharmaceutical compositions of the invention may be prepared in any formulation that is conventionally used in the art. For example, the cosmetic or pharmaceutical compositions may be prepared in the formulation of moisturizers, creams or lotions, milks, ointments, unguents, pomades, liniments or balms, emulsions, microemulsions, fatty ointments, sera, soaps, shampoos, pastes, gels, gel-creams, emulsion-gels, suspensions, foams or mousses, tinctures, solutions, hydroalcoholic solutions, patches, bandages, powders, bars, pencils and sprays or aerosols. In a preferred embodiment, the cosmetic or pharmaceutical composition is formulated as a moisturizer, cream or lotion, milk, ointment, unguent, pomade, liniment or balm, emulsion, serum, soap, shampoo, paste, gel, gel-cream, emulsion-gel, suspension, foam or mousse, tincture, solution, patch, bandage, powder, bar, pencil and spray or aerosol. More preferably, as a moisturizer, cream, pomade or balm.

All these systems are well-known to the person skilled in the art. The manufacture of the topically administrable pharmaceutical or cosmetic compositions of the invention is effected in a manner known per se, for example by mixture of the lysates of M. citrifolia and G. herbaceum, preferably in glycerin, in the selected excipients, preferably in water.

In a preferred embodiment, the cosmetic or pharmaceutical composition of the invention comprises glycerin.

Preferably, the cosmetic or pharmaceutical composition of the invention is for topical application.

In a preferred embodiment, the cosmetic or pharmaceutical composition is for skin administration. In the present disclosure, a cosmetic or pharmaceutical skin composition is intended to define a composition that is for use in the skin.

The present invention also envisions the provision of the compositions in solid form. Such forms may, for example, have the shape of a bar intended for application on the lips. These compositions may further contain from about 1% to about 20% of a suitable thickening agent, such as those already described, and, optionally, emulsifiers and water. In this case, it can be readily understood that the amount of water must be reduced so that the product is in solid form.

Further details concerning suitable topical formulations may be obtained by reference to standard textbooks such as "Harry's Cosmeticology" 9th Edition (2015), Chemical Publishing Co.

In a preferred embodiment, the combination of the lysates described herein, is a synergistic combination within the claimed ranges.

### Cosmetic uses

In a particular embodiment, the composition of the invention is a cosmetic composition. Therefore, an aspect of the invention is directed at the non-therapeutic use of the cosmetic composition to densify dermis, strengthen dermal-epidermal junction, thicken epidermis and/or provide anti-glycation defense means to the skin, or for the non-therapeutic amelioration or the non-therapeutic counteraction of a skin condition selected from dry skin, roughening skin, aging skin, and skin wrinkle.

In a preferred embodiment, the combination of the lysates present in the cosmetic composition of the invention described herein, is a combination with synergistic effects in at least one of the uses disclosed herein, within the claimed ranges.

The cosmetic composition of the invention may be prepared in the formulation of sunscreen cream, moisturizing cream, softening cosmetic water, convergence cosmetic water, nutrition cosmetic water, nutrition cream, massage cream, essence, eye cream, pack, spray, or powder. In a preferred embodiment, the cosmetic composition is in the form of a cream, more preferably a sunscreen cream or a moisturizing cream.

The application or administration frequency may vary widely, depending on the needs of each subject, an application or administration range from once a day to ten times a day being suggested, preferably from once a day to four times a day, more preferably from once a day to three times a day, still more preferably one or two times a day.

Topical application means depositing or spreading the compound and the compositions over the epidermic tissue (including skin and oral, gingival, nasal, etc. tissues).

In use, a small quantity of the composition, for example from 1 to 100 mL, is applied to exposed areas of the skin, from a suitable container or applicator and, if necessary, it is then spread over and/or rubbed into the skin using the hand or fingers or a suitable device.

### Pharmaceutical composition

In an aspect of the invention, the pharmaceutical skin of the invention is for use in the prevention and/or treatment of dermic inflammatory conditions.

The pharmaceutical composition of the invention may be prepared in the formulation of creams, pomades, or balms, preferably pomades or balms.

The application or administration frequency of the pharmaceutical composition may vary widely, depending on the needs of each subject, an application or administration range from once a month to ten times a day being suggested, preferably from once a week to four times a day, more preferably from three times a week to three times a day, still more preferably one or two times a day.

The inventors have surprisingly found that the compositions of the present invention, which comprise a combination of M. citrifolia and G. herbaceum lysates, produce a synergistic effect at least in the reduction of cellular ROS.

In a particular embodiment, the pharmaceutical composition of the invention is for use in the prevention and/or treatment of dermatitis, xerosis, acne, psoriasis, atopia, redness and itch from insect bites, photoaging, acne-like skin, burning or stinging skin sensations, erythema of the skin including erythema multiforme minor, erythema multiforme major and erythema nodosum, cutaneous hyperactivity with dilation of blood vessels of the skin, Lyell's syndrome, Stevens-Johnson syndrome, local itching and discomfort associated with hemorrhoids, hemorrhoids, urticaria, pruritis, purpura, varicose veins, lichen simplex chronicus, pseudofolliculitis barbae, granuloma annulare, actinic keratosis, basal cell carcinoma, squamous cell carcinoma, and eczema.

In a preferred embodiment, the combination of the lysates present in the pharmaceutical composition of the invention described herein, is a combination with synergistic effects in at least one of the uses disclosed herein, within the claimed ranges.

In a particular aspect of the invention, the compositions of the invention are for use in the treatment or amelioration of inflammation conditions, including dermatitis, wherein dermatitis is selected from actinic dermatitis, contact dermatitis, atopic dermatitis, seborrheic dermatitis, carcinomatous dermatitis, diaper dermatitis, stasis dermatitis, neurodermatitis, dermatomyositis and radiation-induced dermatitis.

### EXAMPLES

The present invention will now be described by way of examples which serve to illustrate the construction and testing of illustrative embodiments. However, it is understood that the present invention is not limited in any way to the examples below.

### Example 1. Preparation of the M. citrifolia lysates

*M.citrifolia* plant cells were grown in Murashige & Skoog (MS) medium containing plant hormones: 2 mg/L of naphthaleneacetic acid and 0.2 mg/L of kinetin for 14 days under stirring at 115 rpm, in the dark at a temperature of 25 +/- 2°C.

For the elicitation of the plant cells, the cell suspension was diluted one time with Murashige & Skoog (MS) medium containing 0.5 mg/L of naphthaleneacetic acid and 0.5 mg/L of kinetin with sucrose at a final concentration of 80g/L.

A first composition containing cyclodextrins (such as the ones sold by Wacker, ref. 121877) at a final concentration of 72 g/L is added.

A first composition containing methyl jasmonate at a final concentration of 500 µM is added.

The composition resulting from the previous step is maintained under stirring for 14 days at 115 rpm, in the dark and at a temperature of 25 +/- 2°C.

At this point, the dry weight residue was 80 g/L.

In the present example, glycerin and citric acid monohydrate are then added so that the final concentrations are 68,5% glycerin, 30% cellular composition from step e) and 1,5% citric acid monohydrate.

A final lysis step is performed: the composition obtained in the previous steps was processed in an Ultraturrax T-25 Basic apparatus at a temperature below 30 °C, using a dispersing tool (IKA S 25 N - 25 F). The lysis progress was followed by optic microscopy.

The steps described above provide a 30% lysate of dedifferentiated *M. citrifolia* cells in glycerin, at a density of 1.10-1.30 g/mL. The lysate obtained from the steps above has a biomass concentration of 0.280 g/mL.

### Example 2. Preparation of the G. herbaceum lysates

*G.herbaceum* plant cells were grown in Murashige & Skoog (MS) medium containing plant hormones: 2 mg/L of 2,4-dichlorophenoxyacetic acid and 1 mg/L of kinetin for 7 days under stirring at 115 rpm, in the dark at a temperature of 25 +/- 2°C.

For the elicitation of the plant cells, a first composition containing cyclodextrins (such as the ones sold by Wacker, ref. 121877) at a final concentration of 36 g/L were added.

A first composition containing sucrose at a final concentration of 30g/L was added.

A first composition containing methyl jasmonate at a final concentration of 150 µM was added.

The composition was maintained under stirring for 3 days at 115 rpm, in the dark and at a temperature of 25 +/- 2°C.

A second composition containing methyl jasmonate at a final concentration of 150 µM is added and the conditions of the previous paragraph were repeated for 2 days.

A third composition containing methyl jasmonate at a final concentration of 150 µM was added and the conditions of the previous step repeated for 2 additional days.

At this point, the dry weight residue was 60 g/L.

In the present example, glycerin and citric acid monohydrate were then added, so that the final concentrations were 68,5% glycerin, 30% cellular composition from the previous steps and 1,5% citric acid monohydrate.

A final lysis step was performed: the diluted composition was processed in an Ultraturrax T-25 Basic apparatus at a temperature below 30 °C, using a dispersing tool (IKA S 25 N - 25 F). The lysis progress was followed by optic microscopy.

The steps described above provided a 30% lysate of dedifferentiated *G.herbaceum* cells in glycerin, at a density of 1.10-1.30 g/mL. The lysate obtained from the steps above has a biomass concentration of 0.363 g/mL.

### Example 3. Antioxidant activity of the M. citrifolia lysates

To evaluate the ability of the M. citrifolia lysates to suppress or reduce Reactive Oxygen Species (ROS) formation, a human keratinocyte cell culture was chosen as model. The lysates were pure lysates, i.e., those obtained in Example 1, but excluding the step of diluting with glycerin and citric acid monohydrate.

The marker H₂DFFDA was chosen as reporter.

This evaluation is based on the uptake and intracellular hydrolysis of the H₂DFFDA dye by intracellular esterases to the non-fluorescent derivative, DFF. After entering the cell, the esterases hydrolyze the compound into a nonfluorescent intermediate carboxy-2',7'-dihydrofluorescein, which is retained inside the cell due to the large negative charge of the molecule. Oxidation by ROS causes a further conversion into fluorescent carboxy-2',7'-difluorescein.

To evaluate the antioxidant activity of the lysates, four experimental groups were divised. The first group only contained the keratinocyte cell culture (negative control). The second group contained the keratinocyte cell culture and H₂O₂ as positive control. The third and fourth groups contained the keratinocyte cell culture, H₂O₂, and two concentrations of the lysates (1% and 2%).

After H₂O₂ and lysates incubation, the cells were washed with cold phosphate-buffered saline (PBS), centrifuged and resuspended in 300 µl 1x PBS (100.000 cells/ml). Subsequently, the cells were loaded with H₂DFFDA, at a final concentration of 10 µM and incubated for 30-40 min at 37°C, 5% CO₂. Afterwards, the cells were washed with PBS, and cell fluorescence at 4°C was assessed by flow cytometry (Cytomics FC500-MCL).

The results were measured by flow cytometry and showed that neither the 1% nor the 2% of the M. citrifolia lysates was able to reduce the ROS formation.

This example showed that the M. citrifolia lysates alone do not have antioxidant activity.

### Example 4. Antioxidant activity of the G. herbaceum lysates

The study of Example 3 was repeated but this time the lysates were pure lysates from G. herbaceum, i.e., those obtained in Example 2, but excluding the step of diluting with glycerin and citric acid monohydrate.

The results were measured by flow cytometry and showed that neither the 1% nor the 2% of the G. herbaceum lysates was able to reduce the ROS formation. In fact, the results showed an increase in the ROS production upon addition of the lysates.

This example showed that the G. herbaceum lysates alone do not have antioxidant activity.

### Example 5. Antioxidant activity of the M. citrifolia lysates, when combined with the G. herbaceum lysates

The study of Examples 3 and 4 was repeated but this time the M. citrifolia pure lysates of Example 3 were combined with the pure lysates of G. herbaceum, i.e., those described in Example 4.

The results showed a surprising synergistic increase in the antioxidant activity of the combined lysates except when the concentration was 1% each.

The table below summarizes the results obtained in Examples 3, 4 and in the present example, wherein McL stands for M. citrifolia lysate and GhL stands for G. herbaceum lysate.

| Sample | ROS reduction (%) | |
|---|---|---|
| McL 1% | 6.35 | |
| McL 0.5% | 1.59 | |
| McL 0.1% | 0.51 | |
| GhL 1% | 0 | |
| GhL 0.5% | 20.26 | |
| GhL 0.1% | 0 | Sum of ROS reduction individually (%) |
| McL 0.1% | 19.15 | 0.51 |
| GhL 0.1% | | |
| McL 0.5% | 7.72 | 1.59 |
| GhL 0.1% | | |
| McL 0.1% | 29.42 | 20.77 |
| GhL 0.5% | | |
| McL 0.5% | 46.81 | 21.85 |
| GhL 0.5% | | |
| McL 1% | 0 | 6.35 |
| GhL 1% | | |

## Claims

1. A composition comprising:
- a Morinda citrifolia dedifferentiated cell lysate at a concentration equal to or higher than 0.001% and lower than 1%, by weight ratio relative to the total weight of the composition;
- a Gossypium herbaceum dedifferentiated cell lysate at a concentration equal to or higher than 0.001% and lower than 1%, by weight ratio relative to the total weight of the composition.

2. The composition according to claim 1, wherein the Morinda citrifolia dedifferentiated cell lysates result from the lysis of a Morinda citrifolia dedifferentiated cell culture **characterized by** a dry residue of at least 80 g/L.

3. The composition according to any one of claims 1 or 2, wherein the Gossypium herbaceum dedifferentiated cell lysates result from the lysis of a Gossypium herbaceum dedifferentiated cell culture **characterized by** a dry residue of at least 60 g/L.

4. The composition according to any one of claims 1 to 3, wherein each of the cell lysates is at a concentration comprised between 0.001% and 0.9% by weight ratio relative to the total weight of the composition.

5. The composition according to any one of claims 1 to 4, wherein the dedifferentiated cell lysates comprise biomolecules selected from polyphenols, phytosterols, glucose, fructose, saccharose, furanose, arabitol, myo-inositol, lipids and proteins.

6. The composition according to any one of claims 1 to 5, wherein:
- said Morinda citrifolia dedifferentiated cell lysate is a lysate **characterized by** a biomass concentration of 0.28 g/mL; and/or
- said Gossypium herbaceum dedifferentiated cell lysate is a lysate **characterized by** a biomass concentration of 0.36 g/mL.

7. A cosmetic or pharmaceutical composition comprising the composition according to any one of claims 1 to 6, further comprising a cosmetically or pharmaceutically acceptable excipient.

8. The composition according to any one of claims 1 to 6, or the cosmetic or pharmaceutical composition of claim 7, further comprising glycerin.

9. The composition according to any one of claims 1 to 6, or the cosmetic or pharmaceutical composition of claims 7 or 8, **characterized in that** it is a cosmetic or pharmaceutical skin composition.

10. The cosmetic or pharmaceutical skin composition according to claim 9, formulated as a moisturizer, cream or lotion, milk, ointment, unguent, pomade, liniment or balm, emulsion, serum, soap, shampoo, paste, gel, gel-cream, emulsion-gel, suspension, foam or mousse, tincture, solution, patch, bandage, powder, bar, pencil and spray or aerosol.

11. The cosmetic or pharmaceutical skin composition according to claim 10, wherein the formulation is selected from the group consisting of a moisturizer, a cream a pomade and a balm.

12. The pharmaceutical skin composition according to claims 9 to 11, for use in the prevention and/or treatment of dermic inflammatory conditions.

13. The pharmaceutical skin composition according to claims 9 to 11, for use in the prevention and/or treatment of dermatitis, xerosis, acne, psoriasis, atopia, redness and itch from insect bites, photoaging, acne-like skin, burning or stinging skin sensations, erythema of the skin including erythema multiforme minor, erythema multiforme major and erythema nodosum, cutaneous hyperactivity with dilation of blood vessels of the skin, Lyell's syndrome, Stevens-Johnson syndrome, local itching and discomfort associated with hemorrhoids, hemorrhoids, urticaria, pruritis, purpura, varicose veins, lichen simplex chronicus, pseudofolliculitis barbae, granuloma annulare, actinic keratosis, basal cell carcinoma, squamous cell carcinoma, and eczema.

14. The pharmaceutical skin composition for use according to claim 13, wherein dermatitis is selected from actinic dermatitis, contact dermatitis, atopic dermatitis, seborrheic dermatitis, carcinomatous dermatitis, diaper dermatitis, stasis dermatitis, neurodermatitis, dermatomyositis and radiation-induced dermatitis.

15. Non-therapeutic use of the cosmetic composition of claims 9 to 11 to densify dermis, strengthen dermal-epidermal junction, thicken epidermis and/or provide anti-glycation defense means to the skin, or for the non-therapeutic amelioration or the non-therapeutic counteraction of a skin condition selected from dry skin, roughening skin, aging skin, and skin wrinkle.

## Patentansprüche

1. Zusammensetzung, umfassend:
- ein dedifferenziertes Morinda citrifolia-Zelllysat in einer Konzentration von gleich oder höher als 0,001 % und niedriger als 1 %, bezogen auf das Gewichtsverhältnis relativ zum Gesamtgewicht der Zusammensetzung;
- ein dedifferenziertes Gossypium herbaceum-Zelllysat in einer Konzentration von gleich oder höher als 0,001 % und niedriger als 1 %, bezogen auf das Gewichtsverhältnis relativ zum Gesamtgewicht der Zusammensetzung.

2. Zusammensetzung nach Anspruch 1, wobei die dedifferenzierten Morinda citrifolia-Zelllysate aus der Lyse einer dedifferenzierten Morinda citrifolia-Zellkultur resultieren, **gekennzeichnet durch** einen Trockenrückstand von mindestens 80 g/L.

3. Zusammensetzung nach einem der Ansprüche 1 oder 2, wobei die dedifferenzierten Gossypium herbaceum-Zelllysate aus der Lyse einer dedifferenzierten Gossypium herbaceum-Zellkultur resultieren, **gekennzeichnet durch** einen Trockenrückstand von mindestens 60 g/L.

4. Zusammensetzung nach einem der Ansprüche 1 bis 3, wobei jedes der Zelllysate in einer Konzentration zwischen 0,001 % und 0,9 %, bezogen auf das Gewichtsverhältnis relativ zum Gesamtgewicht der Zusammensetzung, enthalten ist.

5. Zusammensetzung nach einem der Ansprüche 1 bis 4, wobei die dedifferenzierten Zelllysate Biomoleküle umfassen, die aus Polyphenolen, Phytosterolen, Glucose, Fructose, Saccharose, Furanose, Arabitol, Myo-Inositol, Lipiden und Proteinen ausgewählt sind.

6. Zusammensetzung nach einem der Ansprüche 1 bis 5, wobei:
- das dedifferenzierte Morinda citrifolia-Zelllysat ein Lysat ist, das durch eine Biomassenkonzentration von 0,28 g/mL gekennzeichnet ist; und/oder
- das dedifferenzierte Gossypium herbaceum-Zelllysat ein Lysat ist, das durch eine Biomassenkonzentration von 0,36 g/mL gekennzeichnet ist.

7. Kosmetische oder pharmazeutische Zusammensetzung, umfassend die Zusammensetzung nach einem der Ansprüche 1 bis 6, weiterhin umfassend einen kosmetisch oder pharmazeutisch akzeptablen Hilfsstoff.

8. Zusammensetzung nach einem der Ansprüche 1 bis 6 oder kosmetische oder pharmazeutische Zusammensetzung nach Anspruch 7, weiterhin umfassend Glycerin.

9. Zusammensetzung nach einem der Ansprüche 1 bis 6 oder kosmetische oder pharmazeutische Zusammensetzung nach Ansprüchen 7 oder 8, **dadurch gekennzeichnet, dass** sie eine kosmetische oder pharmazeutische Zusammensetzung für die Haut ist.

10. Kosmetische oder pharmazeutische Zusammensetzung für die Haut nach Anspruch 9, formuliert als ein(e) Feuchtigkeitsmittel, Creme oder Lotion, Milch, Wundsalbe, Salbe, Pomade, Einreibemittel oder Balsam, Emulsion, Serum, Seife, Shampoo, Paste, Gel, Gel-Creme, Emulsions-Gel, Suspension, Schaum oder Mousse, Tinktur, Lösung, Pflaster, Verband, Pulver, Riegel, Stift und Spray oder Aerosol.

11. Kosmetische oder pharmazeutische Zusammensetzung für die Haut nach Anspruch 10, wobei die Formulierung ausgewählt ist aus der Gruppe, bestehend aus einem Feuchtigkeitsmittel, einer Creme, einer Pomade und einem Balsam.

12. Pharmazeutische Zusammensetzung für die Haut nach Ansprüchen 9 bis 11 zur Verwendung bei der Prävention und/oder Behandlung von dermatologischen Entzündungszuständen.

13. Pharmazeutische Zusammensetzung für die Haut nach Ansprüchen 9 bis 11 zur Verwendung bei der Prävention und/oder Behandlung von Dermatitis, Xerose, Akne, Psoriasis, Atopie, Rötung und Juckreiz durch Insektenstiche, Lichtalterung, akneartiger Haut, brennenden oder stechenden Hautempfindungen, Erythema der Haut, einschließlich Erythema multiforme minor, Erythema multiforme major und Erythema nodosum, kutaner Überreaktion mit Erweiterung der Blutgefäße der Haut, Lyell-Syndrom, Stevens-Johnson-Syndrom, lokalem Juckreiz und Beschwerden im Zusammenhang mit Hämorrhoiden, Hämorrhoiden, Urtikaria, Pruritis, Purpura, Krampfadern, Lichen simplex chronicus, Pseudofolliculitis barbae, Granuloma annulare, aktinischer Keratose, Basalzellkarzinom, Plattenepithelkarzinom und Ekzemen.

14. Pharmazeutische Zusammensetzung für die Haut zur Verwendung nach Anspruch 13, wobei die Dermatitis ausgewählt ist aus aktinischer Dermatitis, Kontaktdermatitis, atopischer Dermatitis, seborrhoischer Dermatitis, karzinomatöser Dermatitis, Windeldermatitis, Stauungsdermatitis, Neurodermitis, Dermatomyositis und strahleninduzierter Dermatitis.

15. Nicht-therapeutische Verwendung der kosmetischen Zusammensetzung nach Ansprüchen 9 bis 11 zur Verdichtung der Dermis, zur Stärkung der dermalenepidermalen Verbindung, zur Verdickung der Epidermis und/oder zur Bereitstellung von Anti-Glykations-Schutzmitteln auf die Haut oder zur nicht-therapeutischen Verbesserung oder zum nicht-therapeutischen Entgegenwirken eines Hautzustands, ausgewählt aus trockener Haut, rauer Haut, alternder Haut und Hautfalten.

## Revendications

1. Une composition comprenant :
- un lysat cellulaire dédifférencié de Morinda citrifolia à une concentration égale ou supérieure à 0,001 % et inférieure à 1 %, en rapport pondéral par rapport au poids total de la composition ;
- un lysat cellulaire dédifférencié de Gossypium herbaceum à une concentration égale ou supérieure à 0,001 % et inférieure à 1 %, en rapport pondéral par rapport au poids total de la composition.

2. La composition selon la revendication 1, dans laquelle les lysats cellulaires dédifférenciés de Morinda citrifolia résultent de la lyse d'une culture cellulaire dédifférenciée de Morinda citrifolia **caractérisée par** un résidu sec d'au moins 80 g/L.

3. La composition selon l'une quelconque des revendications 1 ou 2, dans laquelle les lysats cellulaires dédifférenciés de Gossypium herbaceum résultent de la lyse d'une culture cellulaire dédifférenciée de Gossypium herbaceum **caractérisée par** un résidu sec d'au moins 60 g/L.

4. La composition selon l'une quelconque des revendications 1 à 3, dans laquelle chacun des lysats cellulaires est à une concentration comprise entre 0,001 % et 0,9 % en poids par rapport au poids total de la composition.

5. La composition selon l'une quelconque des revendications 1 à 4, dans laquelle les lysats cellulaires dédifférenciés comprennent des biomolécules choisies parmi les polyphénols, les phytostérols, le glucose, le fructose, le saccharose, le furanose, l'arabitol, le myo-inositol, les lipides et les protéines.

6. La composition selon l'une quelconque des revendications 1 à 5, dans laquelle :
- ledit lysat cellulaire dédifférencié de Morinda citrifolia est un lysat **caractérisé par** une concentration en biomasse de 0,28 g/mL ; et/ou
- ledit lysat cellulaire dédifférencié de Gossypium herbaceum est un lysat **caractérisé par** une concentration en biomasse de 0,36 g/mL.

7. Une composition cosmétique ou pharmaceutique comprenant la composition selon l'une quelconque des revendications 1 à 6, comprenant en outre un excipient cosmétiquement ou pharmaceutiquement acceptable.

8. La composition selon l'une quelconque des revendications 1 à 6, ou la composition cosmétique ou pharmaceutique selon la revendication 7, comprenant en outre de la glycérine.

9. La composition selon l'une quelconque des revendications 1 à 6, ou la composition cosmétique ou pharmaceutique selon les revendications 7 ou 8, **caractérisée en ce qu'**elle est une composition cosmétique ou pharmaceutique pour la peau.

10. La composition cosmétique ou pharmaceutique pour la peau selon la revendication 9, formulée sous forme de crème hydratante, crème ou lotion, lait, produit d'onction, onguent, pommade, liniment ou baume, émulsion, sérum, savon, shampoing, pâte, gel, gel-crème, émulsion gel, suspension, mousse, teinture, solution, timbre, pansement, poudre, barre, crayon et spray ou aérosol.

11. La composition cosmétique ou pharmaceutique pour la peau selon la revendication 10, dans laquelle la formulation est choisie dans le groupe constitué d'une crème hydratante, d'une crème, d'une pommade et d'un baume.

12. La composition pharmaceutique pour la peau selon les revendications 9 à 11, destinée à être utilisée dans la prévention et/ou le traitement des affections inflammatoires dermiques.

13. La composition pharmaceutique pour la peau selon les revendications 9 à 11, destinée à être utilisée dans la prévention et/ou le traitement des dermatites, des xéroses, de l'acné, du psoriasis, de l'atopie, des rougeurs et des démangeaisons dues aux piqûres d'insectes, du photovieillissement, des peaux acnéiques, des brûlures ou des picotements. sensations cutanées, érythème cutané incluant érythème polymorphe mineur, érythème polymorphe majeur et érythème noueux, hyperactivité cutanée avec dilatation des vaisseaux sanguins de la peau, syndrome de Lyell, syndrome de Stevens-Johnson, démangeaisons locales et inconfort associés aux hémorroïdes, hémorroïdes, urticaire, prurit, purpura, varices, lichen simplex chronique, pseudofolliculite barbae, granulome annulaire, kératose actinique, carcinome basocellulaire, carcinome épidermoïde et eczéma.

14. La composition pharmaceutique cutanée à utiliser selon la revendication 13, dans laquelle la dermatite est sélectionnée parmi la dermatite actinique, la dermatite de contact, la dermatite atopique, la dermatite séborrhéique, la dermatite carcinomateuse, l'érythème fessier, la dermatite de stase, la neurodermatite, la dermatomyosite et la dermatite induite par les radiations.

15. Utilisation non thérapeutique de la composition cosmétique des revendications 9 à 11 pour densifier le derme, renforcer la jonction dermo-épidermique, épaissir l'épiderme et/ou apporter des moyens de défense anti-glycation à la peau, ou pour l'amélioration non thérapeutique ou la lutte non-thérapeutique contre une affection cutanée sélectionnée parmi la peau sèche, la peau rugueuse, le vieillissement cutané et les rides cutanées.
